# EUROPÄISCHE PATENTSCHRIFT

(11) **EP 2 162 274 B1**
(45) Veröffentlichungstag und Bekanntmachung des Hinweises auf die Patenterteilung: **24.10.2012**
(21) Anmeldenummer: 08774614.5
(22) Anmeldetag: 02.07.2008
(51) Int. Cl.: B29C 59/16, B01L 3/00, G01N 33/52, G01N 33/543

(54) **VERFAHREN ZUR HERSTELLUNG EINES ANALYSEELEMENTES UND ANALYSEELEMENT**
METHOD FOR THE PRODUCTION OF AN ANALYTICAL ELEMENT AND ANALYTICAL ELEMENT
PROCÉDÉ DE FABRICATION D'UN ÉLÉMENT D'ANALYSE ET ÉLÉMENT D'ANALYSE

(30) Priorität: 03.07.2007 EP 07111622
(43) Veröffentlichungstag der Anmeldung: 17.03.2010
(73) Patentinhaber: Roche Diagnostics GmbH, 68305 Mannheim (DE); F. Hoffmann-La Roche AG, 4070 Basel (CH)
(72) Erfinder: RÖPER, Josef, K., 67141 Neuhofen (DE); FINKE, Werner, 64683 Einhausen (DE); KOSCHORRECK, Beate, 69198 Schriesheim (DE)
(74) Vertreter: Stößel, Matthias
(86) Internationale Anmeldenummer: PCT/EP2008/058473
(87) Internationale Veröffentlichungsnummer: WO 2009/004017

(56) Entgegenhaltungen:
- EP-A- 0 821 233
- EP-A- 1 593 434
- EP-A- 1 832 874
- GB-A- 2 350 678
- US-A1- 2006 234 269

## Beschreibung

Die Erfindung bezieht sich auf ein Analyseelement mit mindestens einem Testfeld zur Analyse einer flüssigen Probe und auf ein Verfahren zu dessen Herstellung.

Zur Analyse von Flüssigproben, beispielsweise Körperflüssigkeiten wie Blut oder Urin, werden häufig Analysegeräte verwendet, bei denen sich die zu analysierende Probe auf einem Testfeld eines Analyseelements befindet und in dem Testfeld gegebenenfalls mit einer oder mehreren Reagenzien reagiert, bevor sie analysiert wird. Die optische, insbesondere photometrische, und die elektrochemische Auswertung von Analyseelementen stellen die gebräuchlichsten Verfahren zur schnellen Bestimmung der Konzentration von Analyten in Proben dar. Analysesysteme mit Analyseelementen zur Probenanalyse werden allgemein im Bereich der Analytik, der Umweltanalytik und vor allem im Bereich der medizinischen Diagnostik eingesetzt. Insbesondere im Bereich der Blutglukosediagnostik aus Kapillarblut besitzen Analyseelemente, die photometrisch oder elektrochemisch ausgewertet werden, einen großen Stellenwert.

Es gibt verschiedene Formen von Analyseelementen. Bekannt sind zum Beispiel im Wesentlichen quadratische Plättchen, die auch als Slides bezeichnet werden, in deren Mitte sich ein mehrschichtiges Testfeld befindet. Diagnostische Analyseelemente, die streifenförmig ausgebildet sind, werden als Teststreifen bezeichnet. Im Stand der Technik sind Analyseelemente umfassend beschrieben, beispielsweise in den Dokumenten CA 2311496 A1, US 5,846,837 A bzw. EP 0 821 233 A2, US 6,036,919 A oder WO 97/02487.

Bei Kapillarspalt-Testelementen wird die Probenflüssigkeit von einem Probenaufgabeort zu einem davon entfernten Probendetektionsort mit Hilfe kapillarer Kräfte in einem Transportkanal (Kapillarkanal, Kapillarspalt) bewegt, um dort eine Nachweisreaktion einzugehen. Kapillarspalt-Testelemente sind beispielsweise aus CA 2549143 oder aus US 2003/0013147 A1 bekannt. Die Mikrokapillaren tragen eine Innenbeschichtung aus hydrophilen und gegebenenfalls aus hydrophoben Materialien. Durch hydrophile und hydrophobe Oberflächeneigenschaften der mit der Probenflüssigkeit in Kontakt tretenden Materialien kann der Flüssigkeitstransport gesteuert werden.

Weitere im Stand der Technik bekannte Analyseelemente sind Analysebänder mit einer Vielzahl von Testfeldern, die in einer Kassette aufgewickelt zur Verwendung in einem Analysegerät bereitgestellt werden. Solche Kassetten und Analysebänder werden zum Beispiel in den Dokumenten DE 103 32 488 A1, DE 103 43 896 A1, EP 1 424 040 A1, WO 2004/056269 A1 und CA 2506358 A1 beschrieben.

Die vorliegende Erfindung bezieht sich auf Analyseelemente beliebiger Form, insbesondere auf streifenförmige Testelemente (z.B. streifenförmige Kapillarspalt-Testelemente) und auf Analysebänder.

Analyseelemente weisen üblicherweise hydrophile und hydrophobe Bereiche auf. Die Begriffe "hydrophob" und "hydrophil" haben hierin die im Fachgebiet allgemein verstandenen Bedeutungen. Eine hydrophile Oberfläche weist eine gute Benetzbarkeit durch Wasser und eine hydrophobe Oberfläche eine schlechte Benetzbarkeit durch Wasser auf. Die Benetzbarkeit einer Oberfläche (und damit zum Beispiel die Fließgeschwindigkeit in einer mit dieser Oberfläche ausgestatteten Kapillare) lässt sich anhand des Kontaktwinkels α, den Wasser (bzw. eine Wasser enthaltende Probe) mit der Oberfläche bildet, ableiten. Bei der Berührung eines Flüssigkeitstropfens mit einer festen Unterlage können zwei Extremfälle auftreten:
- vollkommene Benetzung: die Adhäsionskräfte sind größer als die Kohäsionskräfte. Deshalb wird sich die Probe auf der Oberfläche des festen Körpers ausbreiten;
- unvollkommene Benetzung: die Adhäsionskräfte sind (wesentlich) kleiner als die Kohäsionskräfte. Deshalb wird sich die Flüssigkeit zu einem kugelförmigen Tropfen zusammenziehen.

Die Benetzbarkeit und folglich zum Beispiel die Fließgeschwindigkeit einer flüssigen Probe in einer Kapillare sind umso größer, je kleiner der Kontaktwinkel α ist. Die Füllzeit zur Füllung einer Kapillare pro Strecke steigt mit dem Kontaktwinkel exponentiell an. Bei Wasser enthaltenden Proben genügt die Angabe des Kontaktwinkels von Wasser, um die materialspezifischen kapillaren Eigenschaften zu charakterisieren. Der Begriff "hydrophobisieren" bedeutet in diesem Zusammenhang eine Veränderung einer Oberfläche, die eine Vergrößerung des Kontaktwinkels bewirkt, denen eine flüssige wasserhaltige Probe mit der Oberfläche bildet.

Als Extremfall hydrophober Oberflächen sind so genannte "superhydrophobe" Oberflächen zu nennen. Derartige Oberflächen sind vollständig unbenetzbar, so dass Wassertropfen von diesen Oberflächen vollständig abperlen. Derartige Oberflächen werden beispielsweise als selbst reinigende Oberflächen eingesetzt.

Im Stand der Technik werden hydrophile oder hydrophobe Oberflächeneigenschaften z.B. von Folien durch Imprägnierung- und/oder Beschichtungsprozesse mit hierfür geeigneten Hilfsstoffen (z.B. Detergenzien oder Wachsen) erzeugt. Beispielsweise werden in der Halbleiterherstellung bei bestimmten Prozessen gezielt hydrophile oder hydrophobe Oberflächen hergestellt, wodurch sich beispielsweise bestimmte Strukturen erzielen lassen.

Auch in einem Grenzgebiet der Halbleitertechnik und der Biologie, nämlich im Bereich der Analytik mittels Halbleiterchips (auch als "Lab-on-a-chip" bezeichnet), werden in vielen Fällen gezielt hydrophilisierte oder hydrophobisierte Oberflächen eingesetzt, um so genannte "Assays" für bestimmte Zielsubstanzen herzustellen. Ein Beispiel derartiger Chips mit funktionalisierten Oberflächen ist in US 2006/0234269 A1 beschrieben. Dabei werden in einer in der Halbleiterherstellung üblichen Schichttechnologie funktionalisierte Vielschichtanordnungen eingesetzt, welche anschließend durch Lichteinwirkung, beispielsweise durch Laserbestrahlung, gezielt modifiziert und teilweise wieder abgetragen werden, um die gewünschte Strukturierung der Funktionalisierung zu erzeugen. Derartige Vielschichtprozesse sind jedoch technisch aufwändig, erfordern in vielen Fällen aufwändige Reinraumtechnik und teure Prozesstechnologie und sind für die Massenherstellung analytischer Testelemente für den täglichen Bedarf daher zumeist nicht geeignet.

EP 0 821 233 A2 beschreibt einen diagnostischen Testträger, enthaltend eine Tragschicht mit einer darauf angeordneten, zur Bestimmung von Analyt in einer flüssigen Probe erforderliche Reagenzien enthaltende Nachweisschicht und ein die Nachweisschicht überdeckendes Netzwerk, das größer ist als die Nachweisschicht und auf der Nachweisschicht befestigt ist. Das Netzwerk ist hydrophil, aber alleine nicht kapillaraktiv und weist eine inerte Abdeckung aus einem probenundurchlässigen Material auf, welches die Probenauftragsstelle frei lässt. Die EP 0 821 233 A2 geht also von benetzbaren Netzwerken aus, die zunächst insgesamt einen möglichen Probenaufgabebereich darstellen. Der eigentliche Probenaufgabebereich wird anschließend beispielsweise durch Abdeckungen in Form von Klebebändern definiert. Derartige Prozesse sind jedoch in der Praxis in der Regel aufwändig, erfordern zahlreiche einzelne Herstellungsschritte und sind in vielen Fällen beispielsweise für eine Massenherstellung mittels eines Rolle-zu-Rolle-Verfahrens nur bedingt geeignet.

US 2001/0024805 A1 bezieht sich auf ein Verfahren zum Durchführen von Analysen, umfassend das Bereitstellen einer Vorrichtung zum Kultivieren von Mikroorganismen. Die Vorrichtung weist eine Auswertungsoberfläche auf, die hydrophile, flüssigkeitszurückhaltende Zonen und eine hydrophobe, erhöhte Fläche zwischen diesen Zonen aufweist. Die hydrophobe Oberfläche kann auf verschiedene Arten hydrophob gemacht werden. Zum Beispiel kann auf einem Polyethylenfilm, der durch Beimischen eines Netzmittels hydrophil gemacht wurde, eine dünne Schicht von acryliertem Silikon oder anderem hydrophoben Material aufgetragen werden.

WO 2005/054845 A1 betrifft ein analytisches Testelement zur Bestimmung eines Analyten in der Flüssigkeit. Das Testelement umfasst einen inerten Träger, eine Aufgabezone für Probenmaterial, eine Nachweiszone zur Bestimmung des Analyten und einen Kanal oder Spalt zum Transport von Flüssigkeit von der Aufgabezone zur Nachweiszone. Das Testelement weist zumindest in einem Bereich um die Aufgabezone eine hydrophob strukturierte Oberfläche auf. Die strukturierte hydrophobe Oberfläche mit Lotus-Effekt wird durch Beschichten, Tränken, Sprühen, Coexdrudieren oder Spritzguss erzeugt.

CA 2506358 A1 hat ein Verfahren zur Herstellung eines Analysebandes für Flüssigproben zum Gegenstand. Dabei wird ein aufrollbares Transportband mit einer Vielzahl von in Bandrichtung im Abstand voneinander befindlichen Testelementen versehen, die als selbstklebende Testetiketten auf dem Transportband angebracht werden. Die Testetiketten enthalten ein doppelseitiges Klebeband und als Testfeld einen Nachweisfilm mit geringer Breite zentriert auf der oberen Klebeschicht des Klebebandes, so dass seitliche Klebestreifen der Klebeschicht freigehalten werden. Darauf ist eine als Gewebe ausgebildete Decklage aufgebracht, die breiter als der Nachweisfilm ist und deren seitlich überstehende Ränder durch die seitlichen Klebestreifen fixiert werden. Die überstehenden Ränder der Decklage außerhalb des Nachweisfilms werden hydrophobisiert durch Bedrucken mit einer wasserabweisenden Imprägnierung, so dass nur eine zentrale Zone über dem Nachweisfilm eine flüssige Probe aufsaugen und zu dem Nachweisfilm transportieren kann.

Diese im Stand der Technik bekannten Verfahren zur Hydrophobisierung haben den Nachteil, dass zur Beschichtung verwendete Hilfsstoffe (z.B. Detergenzien, Thermotransferwachse) über Jahre mit gleich bleibender Qualität und mit zuverlässigen Lieferkonditionen zur Verfügung stehen müssen. Ferner können störende Wechselwirkungen bei gleichzeitiger Verwendung von hydrophilen und hydrophoben Reagenzien auftreten (z.B. Wechselwirkung eines aufgedruckten hydrophoben Thermotransferwachses mit einer Detergensbeschichtung des bedruckten Gewebes). Außerdem können häufig keine scharfen Grenzen zwischen hydrophilen und hydrophob beschichteten Bereichen hergestellt werden.

Weiterhin wird im Stand der Technik die Bestrahlung mit elektromagnetischer Strahlung zur Hydrophobisierung eingesetzt. Gemäß EP 1291173 A1 wird eine hydrophile Schicht aus einer bestimmten wärmeempfindlichen Zusammensetzung hergestellt und durch IR-Strahlung belichtet, wodurch die belichteten Bereiche der Schicht stärker hydrophob werden. Gemäß WO 98/43739 A2 können durch Plasmabehandlungen hydrophil gemachte Oberflächen zurück in eine hydrophobe Oberfläche umgewandelt werden durch Anwendung von Lösungsmitteln, Ultraviolett-Licht oder Wärme. Diese Art der Umwandlung von hydrophilisierten Oberflächen in hydrophobe Oberflächen durch die in WO 98/43739 A2 beschriebenen unspezifischen Maßnahmen ist jedoch in der Praxis mit Nachteilen verbunden. So lässt sich beispielsweise feststellen, dass plasmabehandelte Oberflächen nur temporär ihren hochenergetischen, hydrophilen Zustand halten. Die Oberflächenenergien sind in der Regel erheblich erhöht und somit nicht langzeitstabil. Dies bedeutet jedoch, dass die Oberflächen nach einiger Zeit wieder in ihren hydrophoben Zustand übergehen, was zu einer erheblichen Veränderung der Testelementeigenschaften führen kann. Diese geringe Stabilität der Hydrophilisierung ist in der Regel auch dafür verantwortlich, dass das in WO 98/43739 A2 beschriebene Verfahren wirken kann, denn die offenbarten unspezifischen Prozesse und Einsatzstoffe wie Wärme, Lösungsmittel und UV-Licht würden bei einer stabil hydrophiliserten Oberfläche ansonsten mit hoher Wahrscheinlichkeit keine Überführung in einen hydrophoben Zustand herbeiführen.

US 6 271040 B1 Offenbart ein Verfahren gemäβ dem Oberbegriff des Anspruchs 1 und ein produckt gemäβ dem Oberbegriff des Anspruchs 9.

Weiterhin sind die im Stand der Technik bekannten Verfahren aufwendig und kostenintensiv, da dem Hydrophobisieren vorbereitende Verfahren voraus gehen müssen (Beschichtung mit wärmeempfindlicher Zusammensetzung/Hydrophilisieren durch Plasmabehandlung).

Die Aufgabe der Erfindung besteht darin, die Nachteile des Standes der Technik zu vermeiden. Insbesondere ist es auch Aufgabe der Erfindung, ein Verfahren zur Herstellung eines Analyseelements bereit zu stellen, mit dem Oberflächenbereiche des Analyseelements kostengünstig und flexibel hydrophobisiert werden können. Das Verfahren soll insbesondere für eine großtechnische Herstellung geeignet sein, insbesondere ein Rolle-zu-Rolle-Verfahren.

Diese Aufgabe wird erfindungsgemäß gelöst durch ein Verfahren zur Herstellung eines Analyseelements mit mindestens einem Testfeld zur Analyse einer flüssigen Probe, wobei ein Träger bereitgestellt wird, auf dem ein Polymergewebe angeordnet ist. Zumindest ein Teilbereich des Polymergewebes wird mit UV-Laserlicht bestrahlt und dadurch hydrophobisiert.

Zur Herstellung des Analyseelements wird ein Träger bereitgestellt, auf dem ein Polymergewebe angeordnet ist. Der Träger kann beispielsweise eben-, insbesondere streifen- oder bandförmig, oder dreidimensional geformt sein.

Ein Testfeld ist in diesem Zusammenhang ein Feld, in dem eine flüssige Probe analysiert wird. Das Testfeld ist vorzugsweise auf dem Träger angeordnet. Beispielsweise ist ein Testfeld eine Nachweiszone, die so gestaltet ist, dass sich dort bestimmte Bestandteile der flüssigen Probe oder ihre Reaktion mit in der Nachweiszone vorhandenen Reagenzien nachweisen lassen. Ein Beispiel ist eine Zone, in der eine Nachweisreaktion für Glucose in einer flüssigen Probe (z.B. einer Blutprobe) und deren photometrische Auswertung stattfindet.

Die Erfindung beruht damit auf einer Hydrophobisierung von Geweben, im Gegensatz beispielsweise zu den bekannten Halbleiterprozessen, wie beispielsweise dem in US 2006/0234269 A1 beschrieben Verfahren, bei welchem geschlossene Oberflächen von flüssigkeitsführenden Schichten in Chips, Mikrochips oder Slides durch gezielten Schichtabtrag hydrophobisiert werden. Im Gegensatz zu derartigen Halbleitertechniken, welche üblicherweise eine chargenweise Herstellung (batch-to-batch) bedingen, eignet sich die erfindungsgemäße Verwendung von Geweben erheblich besser für eine Massenherstellung, insbesondere eine Rolle-zu-Rolle-Herstellung.

Im Gegensatz zu bekannten Verfahren, bei welchen ebenfalls mit Netzwerken gearbeitet wird, wie beispielsweise dem in EP 0 821 233 A2 beschriebenen Verfahren, kann jedoch auf Abdecktechniken verzichtet werden, bei welchen die Strukturierung mittels eines Abklebeprozesses durch Folien generiert werden muss.

Das Polymergewebe im Sinne der Erfindung ist ein Gewebe aus Polymerfäden oder ein Vlies aus Polymerfasern. Das Polymergewebe besteht vorzugsweise aus einem Polymer ausgewählt aus der Gruppe bestehend aus Polyester, Polyamid, Polypropylen und Polyacrylnitril.

Vorzugsweise ist das auf dem Träger angeordnete Polymergewebe (vor der Bestrahlung mit UV-Laserlicht) hydrophil oder hydrophilisiert und dient auf dem Analyseelement in (den nicht mit UV-Laserlicht bestrahlten) Teilbereichen zur Aufnahme und/oder zum Transport der flüssigen Probe. Besonders bevorzugt ist das Polymergewebe mit dem Testfeld direkt in Kontakt, z.B. bedeckt es das Testfeld ganz oder teilweise, so dass es in den nicht hydrophobisierten Bereichen eine flüssige Probe aufnehmen und an das Testfeld weiterleiten kann. Das Polymergewebe kann jedoch auch in mindestens einem Teilbereich selbst als Testfeld zur Analyse einer flüssigen Probe dienen und z.B. in diesem mindestens einen Teilbereich Reagenzien zum Nachweis eines Analyten in der flüssigen Probe enthalten. Das Polymergewebe kann ein Detergens zur Spreitung der Probe auf dem Gewebe aufweisen, enthält jedoch vorzugsweise selbst keine Reagenzien zum Nachweis eines Analyten. Diese Reagenzien befinden sich vorzugsweise lediglich in einem Nachweisfilm. Die flüssige Probe ist bevorzugt eine Wasser enthaltende Probe, z.B. Plasma, Blut, intestitielle Flüssigkeit, Urin, Speichel, Schweiß oder eine Probe der Wasseranalytik (insbesondere Altwasser).

Erfindungsgemäß wird zumindest ein Teilbereich des Polymergewebes mit UV-Laserlicht bestrahlt und dadurch in dem bestrahlten Bereich hydrophobisiert. UV-Laserlicht ist das von einem Laser emittierte Licht mit einer Wellenlänge im Bereich von 1 nm bis 380 nm. Bevorzugte Wellenlängen des für das erfindungsgemäße Verfahren verwendeten UV-Laserlichts sind 248 nm, 266 nm und 355 nm. Das UV-Laserlicht wird vorzugsweise durch diodengepumpte Festkörperlaser oder Excimer-Laser bereitgestellt. Vorzugsweise wird zumindest ein Teilbereich des Polymergewebes gezielt mit UV-Laserlicht bestrahlt. Gezielt bedeutete in diesem Zusammenhang, dass keine Masken oder ähnliches verwendet werden, sondern dass mindestens ein Laserstrahl mittels geeigneter optischer Komponenten auf den Teilbereich fokussiert wird und diesen Teilbereich abfährt (scannt), so dass eine ortsaufgelöste Hydrophobisierung des Polymergewebes erreicht wird. Alternativ oder zusätzlich kann jedoch auch ein Maskenverfahren bei der Bestrahlung mit Laserlicht eingesetzt werden, beispielsweise ein Masken-Belichtungsverfahren mit einem UV-Excimerlaser.

Im Gegensatz zu den aus dem Stand der Technik bekannten Verfahren, insbesondere dem in WO 98/43739 A2 offenbarten Verfahren, wird also durch Verwendung des UV-Laserlichtes, im Gegensatz zu unspezifischer Lichtbestrahlung, eine spezifische Behandlung durchgeführt. Durch diese spezifische Behandlung, welche ortsaufgelöst durchgeführt werden kann, können chemisch und langzeitstabile hydrophile Oberflächen mittels spezifischer UV-Laserstrahlung in einen neuen, hydrophoben Zustand überführt werden.

Der mittels UV-Laserlicht hydrophobisierte Teilbereich des Polymergewebes oder ein vollständig hydrophobisiertes Polymergewebe auf dem Analyseelement kann beispielsweise dazu dienen, die flüssige Probe in ihrem Fluss zu verlangsamen oder abzustoppen oder die Benetzung des Teilbereichs durch die flüssige Probe (z.B. bei der Probenaufgabe) zu verhindern. Durch das Hydrophobisieren eines bereitgestellten hydrophilen Polymergewebes in einem oder mehreren Teilbereichen kann ein Hydrophil-Hydrophob-Muster erzeugt werden.

Durch das Bestrahlen mit dem UV-Laserlicht wird das Polymergewebe in dem mit UV-Laserlicht bestrahlten Bereich strukturiert, d.h. die Oberflächenstrukturierung wird durch das Laserlicht verändert. Insbesondere kann die Polymeroberfläche des Polymergewebes durch das Bestrahlen mit Laserlicht aufgeraut werden. Zur Strukturierung wird vorzugsweise ein gepulster Laser verwendet, wobei die Polymeroberfläche in dem Teilbereich mit dem gepulsten Laserstrahl abgescannt wird und durch das Auftreffen der Laserpulse auf die Polymeroberfläche in einem gewissen Abstand zueinander die Polymeroberfläche strukturiert wird. Durch geeignete Wahl der Laserparameter (Wellenlänge, Leistung, Pulsrate etc.) können gezielt Mikrostrukturen erzeugt werden, die hydrophobe Eigenschaften hervorrufen. Durch das Laserlicht entstehen auf der Polymeroberfläche der Fäden oder Fasern des Polymergewebes verschmolzene, runde Strukturen (Erhebungen und Vertiefungen), deren mittlerer Abstand (z.B. von Vertiefung zu Vertiefung) mit dem Begriff "Hatch-Distance" bezeichnet wird.

Durch eine solche Strukturierung können zum Beispiel Teilbereiche der Polymeroberfläche des Polymergewebes so modifiziert werden, dass sie den so genannten "Lotus-Effekt" aufweisen. Dieser Effekt wird beispielsweise in WO 96/04123 A1, WO 00/58410 A1 oder WO 00/58415 A1 beschrieben. Eine solche Oberfläche weist Erhebungen und Vertiefungen auf, wobei der Abstand zwischen den Erhebungen im Bereich zwischen 0,1 bis 200 µm und die Höhe der Erhebungen im Bereich von 0,1 bis 100 µm liegt, und die Erhebungen hydrophob sind.

Ferner kann das Polymergewebe in dem Teilbereich durch das UV-Laserlicht so strukturiert werden, dass in die entstehenden Vertiefungen Fremdatome, vorzugsweise Luftmoleküle, eingelagert werden können, wodurch die Polymeroberfläche hydrophobisiert wird.

Das erfindungsgemäße Verfahren hat den Vorteil, dass bei der Bestrahlung mit UV-Laserlicht eine direkte Wechselwirkung zwischen dem Laserlicht und dem Polymergewebe stattfindet, die den gewünschten hydrophoben Effekt hervorruft. Zusätzliche Hilfsreagenzien sind nicht erforderlich. Aufwendige Fertigungsprozessschritte (Imprägnierungen, Tränkungen, Plasmabehandlungen) zur Vorbereitung der Hydrophobisierung entfallen. Die Bestrahlung mit UV-Laserlicht gewährt weitgehende Freiheit in der Gestaltung der Geometrie hydrophober Bereiche. Die UV-Laserbehandlung ist im Online-Betrieb gut einstell - und kontrollierbar.

Gemäß einer bevorzugten Ausführungsform der vorliegenden Erfindung ist das Polymergewebe ein monophiles Gewebe, das Fäden enthält, die weitgehend parallel oder senkrecht zueinander verlaufen, wobei die parallel zueinander verlaufenden Fäden einen Abstand zwischen 1 µm und 0,5 mm zueinander aufweisen, bevorzugt zwischen 0,1 und 200 µm. Ein monophiles Gewebe ist ein Gewebe, bei dem in Längs- und Querrichtung einzelne Fäden verwebt sind. Monophile Gewebe sind gegenüber herkömmlichen Gewebetypen definierter, z.B. hinsichtlich ihrer Porengröße, der Homogenität, der Luftdurchlässigkeit oder anderer Eigenschaften. Typische Maschenweiten können beispielsweise zwischen 105 und 285 Mikrometern liegen, typische Fadendurchmesser zwischen 42 und 145 Mikrometern, und typische Gewebedicken zwischen 63 und 260 Mikrometern.

Die Prozessierung von Geweben, wie sie im Rahmen der vorliegenden Erfindung vorgeschlagen wird, unterscheidet sich in der Regel grundlegend von der Funktionalisierung geschlossener Oberflächen, wie sie beispielsweise aus der Halbleitertechnik bekannt ist, oder von der Prozessierung von Folien. Während Halbleiterprozesse oder die Funktionalisierung von Folien üblicherweise unter Verwendung von Beschichtungsprozessen (Coating) ablaufen, beispielsweise mittels Spin-Coating oder Rakeln, sind für eine Verarbeitung von Gewebe derartige Techniken nicht anwendbar. Für das erfindungsgemäße Herstellungsverfahren wird ein Träger bereitgestellt, auf dem das Polymergewebe mit einem Detergens beschichtet ist. Dabei ummantelt das Detergens vorzugsweise die einzelnen Fäden oder Fasern des Polymergewebes, so dass die Grenzflächenspannung zwischen der flüssigen Probe und der Oberfläche des Polymergewebes herabgesetzt wird. Das Detergens kann beispielsweise durch einen Tränkungsprozess auf das Polymergewebe aufgebracht werden, im Gegensatz zu den oben beschriebenen, Halbleiter-typischen Beschichtungstechnologien.

Bevorzugt ist das Detergens mindestens ein Detergens ausgewählt aus der Gruppe bestehend aus Natriumdioctylsulfosuccinat (DONS), Mega-8® (Ocatanoyl-N-methylglucamid) und Nonylphenolethoxylaten, insbesondere Polyethylenglycol-[4-(1,1,3,3-tetramethylbutyl)phenyl]-ether (Triton®). Das Detergens, insbesondere das DONS, kann beispielsweise auf ein Polyestergewebe durch Tränken des Gewebes mit in Ethanol gelöstem DONS und anschließendes Trocknen als feste Schicht aufgebracht werden.

Beispielsweise kann mit einem hydrophilen, hydrophoben oder bereits zumindest teilweise hydrophobisierten Polymergewebe begonnen werden. Dieses Gewebe kann dann mit dem Detergens getränkt werden, beispielsweise indem das Gewebe durch eine entsprechende Wanne mit dem Detergens, beispielsweise einer DONS-Lösung, gezogen wird. Auch andere Tränktechniken sind bekannt und können eingesetzt werden, beispielsweise Sprühtechniken oder ähnliches. Durch diese Tränkung mit dem Detergens wird das Gewebe hydrophil, oder die Hydrophilie des Gewebes wird gesteigert.

Dieses Tränken kann auch großtechnisch durchgeführt werden, beispielsweise in einem Rolle-zu-Rolle-Prozess, im Gegensatz zu den aus der Halbleitertechnik bekannten Batch-Prozessen. Auch eine Kombination mit einem Laserprozess ist großtechnisch möglich. Die Erfindung stellt also eine Kombination flächiger Auftragsverfahren auf Gewebe, wie beispielsweise Tränken, mit räumlich aufgelösten Verfahren in Form von Laserstrukturierungen bereit. Alle diese Schritte sind als Rolle-zu-Rolle-Prozesse realisierbar.

Anschließend kann die beschriebene Bestrahlung mit UV-Laserlicht erfolgen, um das derartig hydrophiliserte Gewebe in dem Teilbereich gezielt wieder zu hydrophobisieren. Das Detergens wird in dem mit UV-Laserlicht bestrahlten Teilbereich des Polymergewebes zumindest teilweise durch das UV-Laserlicht entfernt. Die durch UV-Laserbestrahlung hervorgerufene Hydrophobisierung beruht dabei auf einer Entfernung des Detergens von dem Polymergewebe, insbesondere durch Ablation, und gegebenenfalls zusätzlich auf der Strukturierung der Oberfläche des Polymergewebes.

Es hat sich gezeigt, dass sich die erneute Hydrophobisierung des mit dem Detergens getränkten Gewebes in dem Teilbereich insbesondere derart durchführen lässt, dass eine Superhydrophobisierung auftritt. So wird in vielen Fällen nicht nur das Detergens, insbesondere das DONS, von der Gewebeoberfläche ablatiert, sonden es wird, wie dargestellt, auch auf die Oberflächenstruktur der Fäden des Polymergewebes eingewirkt. Eine derartige Superhydrophobisierung, also eine vollständige Unbenetzbarkeit, ist jedoch gerade im Bereich der analytischen Testelemente eine äußerst wünschenswerte Eigenschaft. So kann eine derartige Superhydrophobisierung erforderlich sein für eine Blutbarriereeigenschaft eines Testfeldes. Eine normale Hydrophobie ist diesbezüglich in vielen Fällen nicht ausreichend.

Die gezielte UV-Laserbestrahlung eines mit Detergens beschichteten Polymergewebes hat eine Vielzahl von Vorteilen, z.B. im Vergleich zu der im Stand der Technik bekannten Bedruckung eines solchen mit Detergens beschichteten Polymergewebes mit einer hydrophoben Substanz (insbesondere die Bedruckung eines mit DONS imprägnierten PET-Gewebes mit Thermotransferwachs). Beim bekannten Thermotransferwachsauftrag wird auf einer Folie aufgebrachtes Wachsgemisch, durch Wärme verflüssigt und auf das mit DONS imprägnierte PET-Gewebe aufgedruckt. Hierbei findet eine Vermischung des hydrophilen DONS mit dem hydrophoben Wachsgemisch statt. Voraussetzung für die Erzeugung einer funktionierenden Hydrophobsperre ist hierbei eine genaue Einstellung des Mischungsverhältnisses aus Detergens (DONS) und Wachs. Die Herstellung definierter Grenzen zwischen hydrophobisierten Bereichen mit Wachs und hydrophilen Bereichen ohne Wachs ist nicht möglich, sondern es entstehen Übergangsbereiche. Durch die UV-Laserbestrahlung werden diese Nachteile vermieden. Eine Wechselwirkung von hydrophilen und hydrophoben Reagenzien findet nicht statt. Für die Gestaltung von hydrophilen und hydrophoben Bereichen auf Analyseelementen ergibt sich eine Vielzahl von Möglichkeiten. Weiterhin ergibt sich über einen Zeitraum von mindestens 6 Monaten eine hohe Stabilität der durch die UV-Laserbehandlung erzeugten hydrophoben Eigenschaft.

Eine bevorzugte Ausführungsvariante des erfindungsgemäßen Verfahrens besteht darin, dass das Analyseelement ein Testelement zur Bestimmung eines Analyten in einer flüssigen Probe ist, das eine Aufgabezone für die flüssige Probe umfasst, wobei das Polymergewebe in einem Bereich um die Aufgabezone mit UV-Laserlicht bestrahlt und dadurch hydrophobisiert wird. Das Testelement ist dabei vorzugsweise ein Teststreifen oder ein auf einem Analyseband angeordnetes Testetikett.

Eine Aufgabezone ist in diesem Zusammenhang ein Bereich des Analyseelements, der dazu vorgesehen ist, eine flüssige Probe aufzunehmen, die auf dem Analyseelement transportiert, gemischt, getrennt, mit Reagenzien kontaktiert und/oder anderweitig prozessiert und analysiert wird.

Durch das Hydrophobisieren im Bereich um die Aufgabezone, in der sich zum Beispiel die Öffnung eines Kapillarkanals oder ein hydrophiles Polymergewebe befindet, wird die Aufgabezone klar begrenzt. Beim Auftragen der flüssigen Probe (z.B. von Blut) auf die Aufgabezone wird überschüssige Probenflüssigkeit entweder durch die Aufgabezone aufgenommen (z.B. in einen Kapillarkanal eingesaugt oder durch ein hydrophiles Polymergewebe zu einer Analysezone weiter transportiert) oder tropft von dem hydrophobisierten Bereich ab, so dass nur die Aufgabezone benetzt wird und eine Verschmutzung der Umgebung der Aufgabezone des Analyseelements und eines das Analyseelement aufnehmenden Messgeräts vermieden wird.

Gemäß einer bevorzugten Ausführungsform der vorliegenden Erfindung wird zur Herstellung eines Testelements ein Nachweisfilm unter Freihaltung von Rändern auf den Träger aufgebracht, der Nachweisfilm von dem Polymergewebe überdeckt, wobei das Polymergewebe in seitlichen Bereichen über den Nachweisfilm übersteht und die freien Ränder des Trägers bedeckt, und das Polymergewebe durch Bestrahlen mit UV-Laserlicht in den seitlichen Bereichen hydrophobisiert. Bevorzugt ist der dabei verwendete Träger ein Klebeband, insbesondere ein doppelseitiges Klebeband, mit dem das Testelement als selbstklebendes Testetikett auf ein Transportband übertragen wird. Der Aufbau des Analyseelements entspricht dabei vorzugsweise dem in CA 2506358 A1 beschriebenen Analyseband, mit dem Unterschied, dass das Polymergewebe außerhalb des Nachweisfilms nicht mit einer wasserabweisenden Imprägnierung bedruckt wird, sondern durch Bestrahlen mit UV-Laserlicht in den seitlichen Bereichen hydrophobisiert wird. Auf die CA 2506358 A1 wird daher ausdrücklich Bezug genommen.

Die Erfindung bezieht sich weiterhin auf ein Analyseelement, das nach dem erfindungsgemäßen Verfahren hergestellt wurde und das mindestens ein Testfeld zur Analyse einer flüssigen Probe umfasst, wobei das Analyseelement einen Träger enthält, auf dem ein Polymergewebe angeordnet ist. Zumindest ein Teilbereich des Polymergewebes ist durch Bestrahlung mit UV-Laserlicht hydrophobisiert.

Gemäß einer bevorzugten Ausführungsvariante ist das erfindungsgemäße Analyseelement ein Analyseband mit einer Vielzahl von in Bandrichtung voneinander beabstandeten Testelementen.

Anhand der Zeichnung wird die Erfindung nachstehend näher erläutert.

Es zeigt
- Figur 1: schematisch eine perspektivische Ansicht eines Analyseelements gemäß dem Stand der Technik in Form eines Analysebandes,
- Figur 2: schematisch eine perspektivische Ansicht eines erfindungsgemäßen Analyseelements in Form eines Analysebandes, hergestellt nach dem erfindungsgemäßen Verfahren,
- Figuren 3A und 3B: eine vergrößerte Ansicht eines durch UV-Laserbestrahlung hydrophobisierten Polymergewebes vor der Laserbehandlung (Figur 3A) und nach der Laserbehandlung (Figur 3B), und
- Figur 4: einen schematischen Ablaufplan eines Ausführungsbeispiels eines erfindungsgemäßen Herstellungsverfahrens.

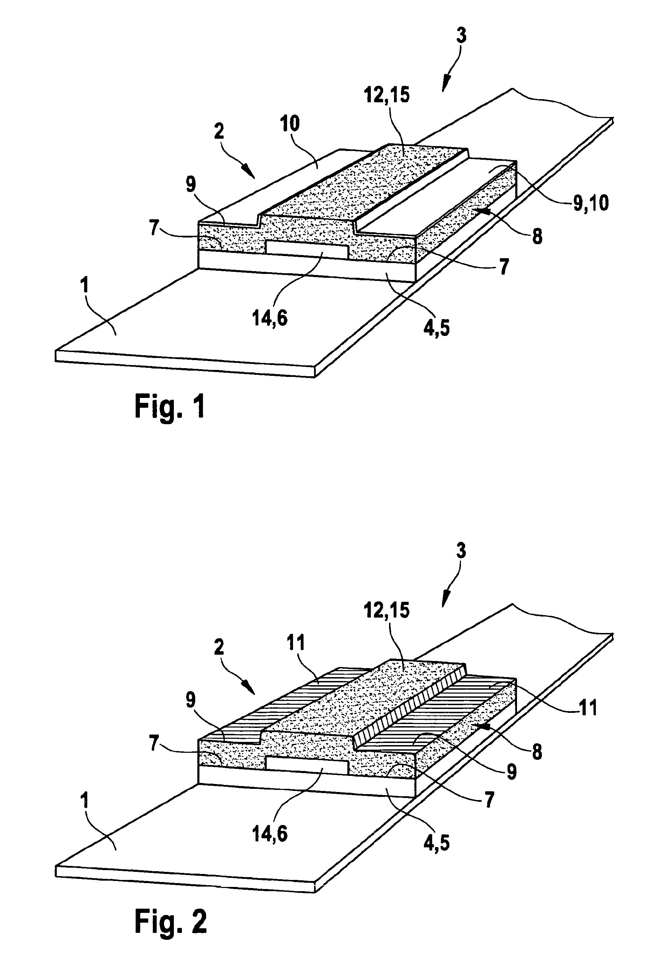

Figur 1 zeigt ein im Stand der Technik bekanntes Analyseelement. Das Analyseelement ist ein in Figur 1 ausschnittsweise dargestelltes Analyseband 3, das ein aufrollbares Transportband 1 und eine Vielzahl von darauf angeordneten, in Bandrichtung voneinander beabstandeten Testelementen 2 umfasst. Die Testelemente 2 (von denen nur eines in Figur 1 dargestellt ist) sind zum Beispiel zur Analyse von Körperflüssigkeiten, insbesondere von Blut, vorgesehen.

Das Testelement 2 ist mehrlagig als selbstklebendes Testetikett aufgebaut. Als Träger 4 des Testelements 2 dient ein doppelseitiges Klebeband 5. Auf der oberen Klebeschicht des doppelseitigen Klebebandes 5 ist ein Nachweisfilm 6 mit geringerer Breite zentriert aufgeklebt, so dass seitliche Ränder 7 auf dem Träger freigehalten werden. Der Nachweisfilm 6 wird von einem Polymergewebe 8 überdeckt. Das Polymergewebe 8 ist breiter als der Nachweisfilm 6, so dass das Polymergewebe 8 in seitlichen Bereichen 9 über den Nachweisfilm 6 übersteht. In den seitlichen Bereichen 9 wird das Polymergewebe 8 daher durch die Ränder 7 des Klebebandes 5 fixiert.

Im Stand der Technik sind die seitlichen Bereiche 9 mit hydrophoben Thermotransferwachs 10 als wasserabweisende imprägnierung bedruckt, so dass nur das zentrale Testfeld (Nachweiszone 14) die aufzubringende flüssige Probe aufsaugen und begrenzt ausbreiten kann. Das Polymergewebe 8 enthält ein Detergens, das sich bei der Imprägnierung mit Thermotransferwachs 10 mit diesem vermischt. Dabei muss eine kritische Balance zwischen Thermotransferwachs 10 und Detergens eingehalten werden, um die gewünschte Hydrophobizität der seitlichen Bereiche 9 einzustellen.

Figur 2 zeigt ein erfindungsgemäßes Analyseelement in Form eines Analysebandes, hergestellt nach dem erfindungsgemäßen Verfahren.

Das Analyseelement ist im Wesentlichen wie das Analyseelement gemäß Figur 1 aufgebaut. Gleiche Bezugszeichen bezeichnen gleiche Bestandteile dieses Analysebandes 3. Im Unterschied zu dem Analyseelement gemäß Figur 1 weist das erfindungsgemäße Analyseelement gemäß Figur 2 jedoch keine Bedruckung aus Thermotransferwachs auf. Stattdessen sind die seitlichen Bereiche 9 des mit Detergens imprägnierten Polymergewebes 8 durch Bestrahlen mit UV-Laserlicht hydrophobisiert oder sogar superhydrophobisiert. Die Funktion dieser beidseitig angeordneten hydrophoben Bereiche 11 ist es, eine lokalisierte Probenauftragung auf das als Aufgabezone 15 vorgesehene mittig angeordnete Polymergewebe 12 zu ermöglichen, das hydrophil ausgebildet ist, ohne eine Kontamination der Umgebung mit der flüssigen Probe, die die seitlichen hydrophoben Bereiche 11 nicht oder nur schlecht benetzt.

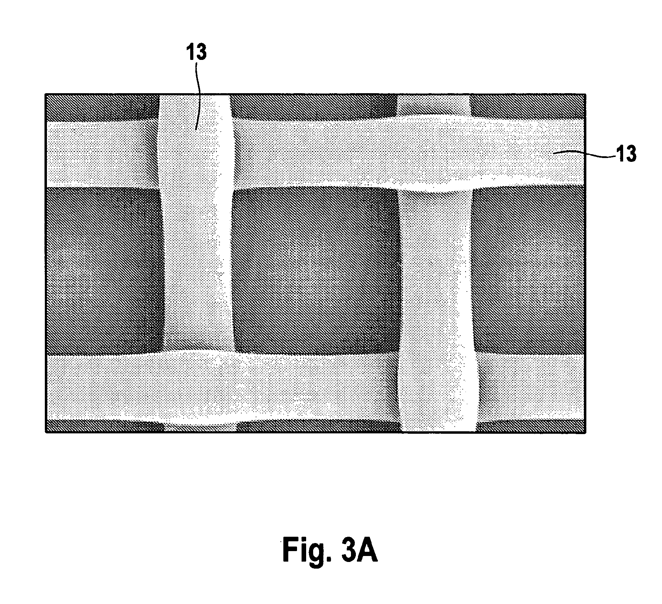

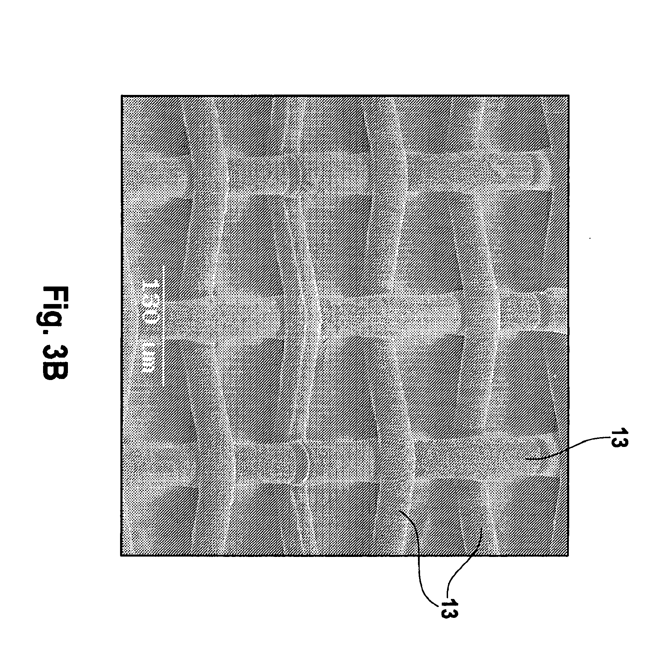

In den Figuren 3A und 3B ist eine vergrößerte Ansicht eines durch UV-Laserbestrahlung hydrophobisierten Polymergewebes dargestellt. Dabei zeigt die Figur 3A ein Polymergewebe vor der Laserbehandlung, wohingegen Figur 3B das Gewebe nach der Laserbehandlung zeigt. Im Vergleich der beiden Bilder ist deutlich zu erkennen, dass die Laserbehandlung eine Oberflächenrauhigkeit erzeugt, welche sich auf die Benetzungseigenschaften der Oberflächen auswirkt.

Das Polymergewebe ist ein Polyestergewebe. Es ist ein monophiles Gewebe, das Fäden 13 enthält, die weitgehend parallel oder senkrecht zueinander verlaufen, wobei die parallel zueinander verlaufenden Fäden 13 einen Abstand von ca. 80-120 µm.

Die Laserbestrahlung erfolgte unter Einsatz verschiedener UV-Lasertypen. So wurde ein eines Excimer-Laser mit einer Wellenlänge von 248 nm, einer Frequenz von 100 Hz, einer Pulsenergie von 7 mJ und einer Spot Size von 400 Mikrometern eingesetzt. Die Anzahl der Pulse wurde in drei Experimenten variiert zwischen 10 Pulsen, 15 Pulsen und 20 Pulsen. Weiterhin wurde ein 4-f Diodenlaser mit einer Wellenlänge von 266 nm eingesetzt. Der Diodenlaser wurde ebenfalls gepulst betrieben, mit einer Pulsfrequenz von 30 kHz und einer Pulsenergie von 10 Mikrojoule. Die Spot Size betrug 18 Mikrometer.

Das Polymergewebe wurde in diesem Fall gemäß der obigen Beschreibung mit DONS imprägniert. Die Laserbehandlung erzeugte eine feine Strukturierung an den Gewebefäden, verbunden mit einer lokalen Entfernung des DONS. Diese Strukturierung ist in Figur 3B deutlich in Form einer Riffelung der Fäden 13 zu erkennen. Dabei erfolgte eine Kontaktwinkelveränderung hin in Richtung zum superhydrophoben Bereich, also in einen Bereich mit einem stehenden Tropfen. Allgemein ließen sich die hydrophoben Eigenschaften bzw. hydrophoben Funktionen eines derartig mit einem UV-Laser behandelten Gewebes in Tests mit Blut und oder einer Kochsalzlösung nachweisen. Die erfindungsgemäß behandelten Gewebe zeigten beispielsweise im Vergleich zu Geweben, die auf konventionelle Weise hydrophobisiert waren, beispielsweise durch den Einsatz von Wachsen, mindestens gleichwertige oder erhöhte hydrophobe Eigenschaften, bis hin zu stehenden, kugelförmiger Tropfen auf dem erfindungsgemäß behandelten Gewebe.

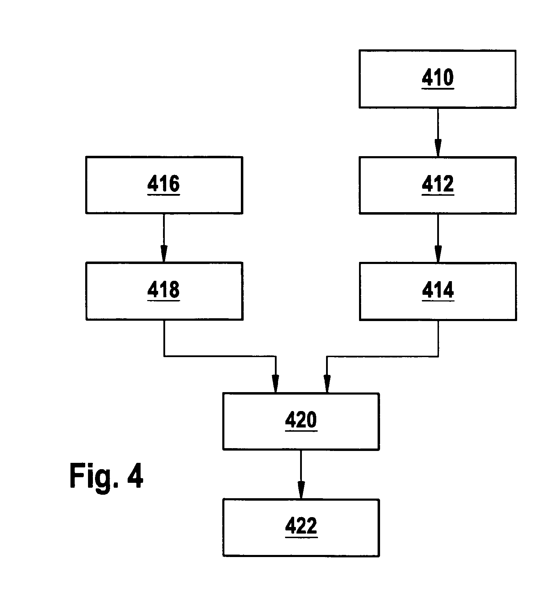

In Figur 4 ist ein schematischer Ablaufplan eines Ausführungsbeispiels eines erfindungsgemäßen Herstellungsverfahrens zur Erzeugung eines Testelements dargestellt. Die dargestellten Verfahrensschritte können durch weitere, in Figur 4 nicht gezeigte Verfahrensschritte ergänzt werden. Weiterhin ist die dargestellte Reihenfolge zwar bevorzugt, jedoch nicht zwingend erforderlich, und es können beispielsweise auch einzelne oder mehrere Verfahrensschritte zusammengefasst, wiederholt oder zeitlich parallel durchgeführt werden. Das Verfahren kann beispielsweise zur Herstellung des in Figur 2 gezeigten Testelements 2 eingesetzt werden, so dass für die einzelnen Komponenten und deren Funktion beispielsweise auf die Beschreibung dieser Figur verwiesen werden kann.

In einem ersten Verfahrensschritt 410 wird ein Transportband 1 bereitgestellt. Auf dieses Transportband 1 wird dann in einem nächsten Verfahrensschritt 412 der Träger 4 und das doppelseitige Klebeband 5 aufgebracht. Auf das derart vorbereitete Transportband 1 bzw. den Träger 4 und die Klebeschicht 5 kann dann in einem Verfahrensschritt 414 das Testfeld 14 in Form des Nachweisfilms 6 aufgebracht werden.

Parallel dazu kann in einem Verfahrensschritt 416 das Polymergewebe 8 bereitgestellt werden. Dieses Polymergewebe 8 wird dann im Verfahrensschritt 418 mit einem Detergens, beispielsweise DONS gemäß der obigen Beschreibung, getränkt.

In Verfahrensschritt 420 wird das derart getränkte Polymergewebe 8 auf das in Verfahrensschritt 414 vorbereitete Testelement aufgebracht, so dass dieses Polymergewebe 8, wie in Figur 2 erkennbar, den Träger 4 und den Nachweisfilm 6 bedeckt.

Anschließend wird in Verfahrensschritt 422 das derart vorbereitete Testelement 2 einer UV-Laserbehandlung in den in Figur 2 mit der Bezugsziffer 11 bezeichneten Bereichen unterzogen. Bei dieser UV-Laserbehandlung in Schritt 422 wird in den Bereichen 11 das Detergens von dem Polymergewebe 8 entfernt und, zusätzlich, die Oberfläche dieses Polymergewebes 8 modifiziert, wie anhand der vorhergehenden Figuren 3A und 3B demonstriert. Hierdurch entstehen die hydrophoben Bereiche 11, welche insbesondere bei Verwendung von DONS und der UV-Laserbehandlung, in den meisten Fällen sogar eine Superhydrophobie aufweisen. Auf diese Weise wird das mittig angeordnete Polymergewebe 12 zur begrenzenden Fläche für den Auftrag von wässrigen flüssigen Proben, wie beispielsweise Blutproben. Die superhydrophoben Bereiche 11 begrenzen somit das Testfeld bzw. die Nachweiszone 14 effektiv.

Es sei darauf hingewiesen, dass auch alternative Herstellungsverfahren zu dem in Figur 4 gezeigten Herstellungsverfahren möglich sind. So ist es beispielsweise möglich, zunächst das mit dem Detergens getränkte Polymergewebe 8 auf den Träger 4 mit dem darauf aufgebrachten Nachweisfilm 6 aufzubringen und dann entsprechende Felder aus dem derart beschichteten Träger auszuschneiden, die dann erst auf das Transportband 1 aufgebracht, beispielsweise aufgeklebt, werden. Die UV-Laserbehandlung kann vor oder nach dem Aufbringen auf das Transportband 1 erfolgen. Auch andere Herstellungsverfahren sind grundsätzlich denkbar.

### Bezugszeichenliste

- 1: Transportband
- 2: Testelement
- 3: Analyseband
- 4: Träger
- 5: doppelseitiges Klebeband
- 6: Nachweisfilm
- 7: Ränder
- 8: Polymergewebe
- 9: seitliche Bereiche
- 10: Thermotransferwachs
- 11: Hydrophobe Bereiche
- 12: mittig angeordnetes Polymergewebe
- 13: Fäden
- 14: Testfeld/Nachweiszone
- 15: Aufgabezone
- 410: Bereitstellen Transportband
- 412: Aufbringen Träger und doppelseitiges Klebeband
- 414: Aufbringen Nachweisfilm
- 416: Bereitstellen Polymergewebe
- 418: Tränken des Polymergewebes mit Detergens
- 420: Aufbringen getränktes Polymergewebe
- 422: UV-Laserbehandlung

## Patentansprüche

1. Verfahren zur Herstellung eines Analyseelements mit mindestens einem Testfeld (14) zur Analyse einer flüssigen Probe, wobei ein Träger (4) bereitgestellt wird, auf dem ein Polymergewebe (8) angeordnet ist, **dadurch gekennzeichnet, dass** ein Träger bereitgestellt wird, auf dem das Polymergewebe (8) mit einem Detergens beschichtet ist, wobei zumindest ein Teilbereich (9) des Polymergewebes (8) mit UV-Laserlicht bestrahlt und dadurch hydrophobisiert wird, wobei das Detergens in dem mit UV-Laserlicht bestrahlten Teilbereich (9, 11) des Polymergewebes (8) zumindest teilweise durch das UV-Laserlicht entfernt wird.

2. Verfahren gemäß Anspruch 1, **dadurch gekennzeichnet, dass** das Polymergewebe (8) ein monophiles Gewebe ist, das Fäden (13) enthält, die weitgehend parallel oder senkrecht zueinander verlaufen, wobei die parallel zueinander verlaufenden Fäden (13) einen Abstand zwischen 1 µm und 0,5 mm zueinander aufweisen.

3. Verfahren gemäß einem der vorhergehenden Ansprüche, **dadurch gekennzeichnet, dass** das Detergens mindestens ein Detergens ausgewählt aus der Gruppe bestehend aus Natriumdioctylsulfosuccinat (DONS), Ocatanoyl-N-methylglucamid und Nonylphenolethoxylaten, insbesondere Polyethylenglycol-[4-(1,1,3,3-tetramethylbutyl)phenyl]-ether.

4. Verfahren nach einem der vorhergehenden Ansprüche, wobei in den Bereichen, in welchen das Detergens mit UV-Laserlicht entfernt wird, eine Superhydrophobisierung des Polymergewebes (8) erfolgt.

5. Verfahren gemäß einem der vorhergehenden Ansprüche, **dadurch gekennzeichnet, dass** das Analyseelement ein Testelement (2) zur Bestimmung eines Analyten in einer flüssigen Probe ist, das einer Aufgabezone (15) für die flüssige Probe umfasst, wobei das Polymergewebe (8) in einem Bereich (9) um die Aufgabezone (15) mit UV-Laserlicht bestrahlt und dadurch hydrophobisiert wird.

6. Verfahren gemäß einem der vorhergehenden Ansprüche, **dadurch gekennzeichnet, dass** zur Herstellung eines Testelements (2) ein Nachweisfilm (6) unter Freihaltung von Rändern (7) auf den Träger (4) aufgebracht wird, der Nachweisfilm (6) von dem Polymergewebe (8) überdeckt wird, wobei das Polymergewebe (8) in seidichen Bereichen (9) über den Nachweisfilm (6) übersteht und die freien Ränder (7) des Trägers (4) bedeckt, und das Polymergewebe (8) durch Bestrahlung mit UV-Laserlicht in den seitlichen Bereichen (9) hydrophobisiert wird.

7. Verfahren gemäß dem vorhergehenden Anspruch, **dadurch gekennzeichnet, dass** der Träger (4) ein Klebeband (5) ist, mit dem das Testelement (2) als selbstklebendes Testetikett auf ein Transportband (1) übertragen wird.

8. Verfahren nach einem der vorhergehenden Ansprüche, wobei ein Rolle-zu-Rolle-Prozess verwendet wird.

9. Analyseelement, herstellbar nach einem Verfahren gemäß einem der vorhergehenden Ansprüche, umfassend mindestens ein Testfeld (14) zur Analyse einer flüssigen Probe, wobei das Analyseelement einen Träger (4) enthält, auf dem ein Polymergewebe (8) angeordnet ist, **dadurch gekennzeichnet, dass** zumindest ein Teilbereich (9, 11) des Polymergewebes (8) durch Bestrahlung mit UV-Laserlicht hydrophobisiert ist, wobei das Polymergewebe (8) auf dem Träger (4) mit dem Detergens beschichtet ist, wobei das Detergens in dem mit UV-Laserlicht bestrahlten Teilbereich (9, 11) des Polymergewebes (8) teilweise durch das UV-Laserlicht entfernt ist.

10. Analyseelement gemäß dem vorhergehenden Anspruch, **dadurch gekennzeichnet, dass** das Analyseelement ein Analyseband (3) mit einer Vielzahl von in Bandrichtung voneinander beabstandeten Testelementen (2) ist.

## Claims

1. Method for producing an analytical element with at least one test field (14) for analyzing a liquid sample, wherein provision is made for a carrier (4) on which a polymer fabric (8) is arranged, **characterized in that** provision is made for a carrier on which the polymer fabric (8) is coated with a detergent, wherein at least one portion (9) of the polymer fabric (8) is irradiated with UV laser light and thereby hydrophobized, wherein the detergent is at least partly removed by the UV laser light in the portion (9, 11) of the polymer fabric (8) irradiated with UV laser light.

2. Method according to Claim 1, **characterized in that** the polymer fabric (8) is a monophilic fabric comprising threads (13) which largely run parallel or perpendicular to one another, wherein the threads (13) running parallel to one another have a spacing of between 1 *µ*m and 0.5 mm to one another.

3. Method according to one of the preceding claims, **characterized in that** the detergent is at least one detergent selected from the group comprising sodium dioctylsulfosuccinate (DONS), octanoyl-N-methylglucamide and nonylphenol ethoxylates, in particular polyethylene glycol [4-(1,1,3,3-tetramethylbutyl)phenyl] ether.

4. Method according to one of the preceding claims, wherein there is a super-hydrophobization of the polymer fabric (8) in those regions in which the detergent is removed by UV laser light.

5. Method according to one of the preceding claims, **characterized in that** the analytical element is a test element (2) for determining an analyte in a liquid sample, which test element comprises an application zone (15) for the liquid sample, wherein the polymer fabric (8) is irradiated with UV laser light in a region (9) around the application zone (15) and thereby hydrophobized.

6. Method according to one of the preceding claims, **characterized in that**, in order to produce a test element (2), a detection film (6) is applied to the carrier (4) whilst keeping edges (7) uncovered, the detection film (6) is covered by the polymer fabric (8), with the polymer fabric (8) projecting beyond the detection film (6) in lateral regions (9) and covering the uncovered edges (7) of the carrier (4), and the polymer fabric (8) is hydrophobized in the lateral regions (9) by irradiation with UV laser light.

7. Method according to the preceding claim, **characterized in that** the carrier (4) is an adhesive tape (5) by means of which the test element (2) is transferred onto a transportation tape (1) as a self-adhesive test label.

8. Method according to one of the preceding claims, wherein a roll-to-roll process is used.

9. Analytical element produced as per a method according to one of the preceding claims, comprising at least one test field (14) for analyzing a liquid sample, wherein the analytical element comprises a carrier (4) on which a polymer fabric (8) is arranged, **characterized in that** at least one portion (9, 11) of the polymer fabric (8) is hydrophobized by irradiation with UV laser light, with the polymer fabric (8) on the carrier (4) being coated with the detergent, wherein the detergent is partly removed by the UV laser light in the portion (9, 11) of the polymer fabric (8) irradiated with UV laser light.

10. Analytical element according to the preceding claim, **characterized in that** the analytical element is an analytical tape (3) with a multiplicity of test elements (2) spaced apart in the direction of the tape.

## Revendications

1. Procédé de fabrication d'un élément d'analyse, avec au moins un champ de test (14) pour l'analyse d'un échantillon liquide, un support (4) étant mis à disposition sur lequel est disposé un tissu polymère (8), **caractérisé en ce qu'**il est mis à disposition un support sur lequel le tissu polymère (8) est revêtu d'un détergent, alors qu'on irradie au moins une zone partielle (9) du tissu polymère (8) avec une lumière à laser UV, ce qui a pour effet de l'imperméabiliser, dans la zone partielle (9, 11) du tissu polymère (8) qui est irradiée avec la lumière à laser UV, le détergent étant éliminé au moins partiellement par la lumière à laser UV.

2. Procédé selon la revendication 1, **caractérisé en ce que** le tissu polymère (8) est un tissu monofil qui contient des fils (13) qui s'étendent amplement parallèlement ou perpendiculairement les uns aux autres, les fils (13) s'étendant parallèlement les uns aux autres présentant un écart mutuel compris entre 1 *µ*m et 0,5 mm.

3. Procédé selon l'une quelconque des revendications précédentes, **caractérisé en ce que** le détergent est au moins un détergent choisi dans le groupe comprenant le dioctylsulfosuccinate de sodium (DONS), le glucamide d'ocatanoyl-N-méthyle et les éthoxylates de nonylphénol, notamment de l'éther de polyéthylenglycol-[4-(1,1,3,3-tétraméthylbutyl)phényle].

4. Procédé selon l'une quelconque des revendications précédentes, où dans les zones dans lesquelles on retire le détergent à l'aide de lumière à laser UV, il s'effectue une super-imperméabilisation du tissu polymère (8).

5. Procédé selon l'une quelconque des revendications précédentes, **caractérisé en ce que** l'élément d'analyse est un élément de test (2) pour la détection d'une analyte dans un échantillon liquide, qui comprend une zone de dosage (15) pour l'échantillon liquide, où dans une zone (9) autour de la zone de dosage (15), on irradie le tissu polymère (8) avec de la lumière à laser UV et on l'imperméabilise de ce fait.

6. Procédé selon l'une quelconque des revendications précédentes, **caractérisé en ce que** pour la fabrication d'un élément de test (2) on applique sur le support (4) un film témoin (6) en laissant libres des bords (7), on recouvre le film témoin (6) avec le tissu polymère (8) dans des zones latérales (9), le tissu polymère (8) débordant par-dessus le film témoin (6) et recouvrant les bords libres (7) du support (4) et le tissu polymère (8) étant imperméabilisé dans des zones latérales (9) par irradiation à la lumière à laser UV.

7. Procédé selon la revendication précédente, **caractérisé en ce que** le support (4) est un ruban adhésif (5) à l'aide duquel on transfère l'élément de test (2) en tant qu'étiquette autocollante sur une bande de transport (1).

8. Procédé selon l'une quelconque des revendications précédentes, un processus rouleau à rouleau étant utilisé.

9. Elément d'analyse, pouvant être fabriqué d'après un procédé selon l'une quelconque des revendications précédentes, comprenant au moins un champ de test (14) pour l'analyse d'un échantillon liquide, l'élément d'analyse contenant un support (4) sur lequel est disposé un tissu polymère (8), **caractérisé en ce qu'**au moins une zone partielle (9, 11) du tissu polymère (8) est imperméabilisée par irradiation avec de la lumière à laser UV, le tissu polymère (8) sur le support (4) étant revêtu avec le détergent, dans la zone partielle (9, 11) du tissu polymère (8) qui est irradiée avec la lumière à laser UV, le détergent étant partiellement retiré par la lumière à laser UV.

10. Elément d'analyse selon la revendication précédente, **caractérisé en ce que** l'élément d'analyse est une bande d'analyse (3) avec une pluralité d'éléments de test (2) écartés les uns des autres en direction de la bande.
